# EUROPEAN PATENT SPECIFICATION

(11) **EP 3 581 930 B1**
(45) Date of publication and mention of the grant of the patent: **03.06.2026**
(21) Application number: 18751798.2
(22) Date of filing: 08.02.2018
(51) Int. Cl.: G01N 33/53, C12M 1/00, G01N 33/543, G01N 37/00, B01J 19/00

(54) **CARRIER FOR IMMOBILIZING BIO-RELATED MOLECULES**
TRÄGER ZUR IMMOBILISIERUNG VON BIOBEZOGENEN MOLEKÜLEN
TRANSPORTEUR POUR IMMOBILISATION DE MOLÉCULES DE TYPE BIOLOGIQUE

(30) Priority: 08.02.2017 JP 2017021269
(43) Date of publication of application: 18.12.2019
(73) Proprietor: Toyo Seikan Group Holdings, Ltd., Shinagawa-ku Tokyo 141-8627 (JP)
(72) Inventor: AKUZAWA, Norio, Yokohama-shi Kanagawa 240-0062 (JP); KASHIWABARA, Ken, Yokohama-shi Kanagawa 240-0062 (JP); NAKAJIMA, Kazuki, Yokohama-shi Kanagawa 240-0062 (JP); JUMYO, Shinya, Yokohama-shi Kanagawa 240-0062 (JP); SARUWATARI, Yoshihiro, Tokyo 141-8627 (JP); ISSHIKI, Atsunori, Yokohama-shi Kanagawa 240-0062 (JP)
(74) Representative: Marks & Clerk LLP
(86) International application number: PCT/JP2018/004453
(87) International publication number: WO 2018/147383

(56) References cited:
- WO-A1-2005/010525
- WO-A1-2011/030823
- JP-A- 2002 211 954
- JP-A- 2003 139 705
- JP-A- 2005 043 312
- JP-A- 2008 249 416
- JP-A- 2008 249 416
- US-A1- 2005 176 003
- US-A1- 2006 204 959
- US-A1- 2012 171 503
- US-A1- 2014 336 081
- US-A1- 2016 216 254

## Description

### Technical Field

The present invention relates to a carrier for bio-related molecule immobilization, and a method of producing the same.

### Background Art

With a demand for safety and soundness of environments and foods, development of techniques for controlling microbial contamination in environmental samples, food ingredients, or products is in progress. As a means for achieving these objects, a method of detecting bio-related molecules such as nucleic acids derived from microorganisms is advantageous in terms of detection sensitivity, specificity, and the like, and development has been carried out for various carriers such as microarrays and DNA chips, in which bio-related molecules are immobilized on a surface-treated substrate. In these carriers, precise spotting apparatuses are used to spot multiple solutions containing different bio-related molecules individually on the substrate in small spots.

Many carriers have been developed as the above-described carriers for bio-related molecule immobilization, including a carrier in which surface treatment of a substrate is improved by controlling the line average roughness of the substrate surface in a predetermined range (Japanese Patent No. 4689475), a carrier whose detection sensitivity is improved by using a highly transparent substrate (Japanese Patent No. 4903518), and the like. Other examples include: US 2012/171503 which refers to a solid support for maintaining a uniform spot shape while improving the capacity for immobilization of nucleic acids upon spotting of nucleic acids onto a solid support is provided; WO 2005/010525 which relates to improved storage safety of a solid support having an active ester group as a functional group capable of forming a covalent bond with a nucleic acid molecule or a protein on a substrate.; JP 2008249416 which relates to an easily-handleable board for a microarray capable of detecting fluorescence in a short time, to which discrimination information can be imparted.; US 2005/176003 which provides a plastic substrate for a microarray chip, which is characterized in that an aminoalkylsilane with an aldehyde group derived from glutaraldehyde and introduced onto an amino group of the aminoalkylsilane exists on a surface of the plastic substrate, and also to a process for its production and a method of its use; US 2006/204959 relating to a solid support capable of immobilizing nucleic acid molecules in a high proportion, and with a high bond strength to nucleic acid molecules.; US 2014/336081 which provides a substrate for biochips which has a high probe loading amounts and a uniform immobilization density, and which further has a high detection sensitivity and a high reproducibility; US 2016/216254 which relates to a substrate for biochips, in which contamination of a biologically relevant substance(s) to be immobilized does not occur. However, particularly in the case of using a resin substrate, there are still problems such as a high background value at the time of detection and poor detection sensitivity of bio-related molecules.

Meanwhile, as the carrier for bio-related molecule immobilization, comprehensive improvement is required for various characteristics such as spotting performance in addition to detection sensitivity. Therefore, there is need for further technical development.

### Summary

The present invention aims to provide a carrier for bio-related molecule immobilization using a resin substrate, which improves in the detection sensitivity while maintaining a good spotting characteristic.

The inventors of the present invention have found that the above-described problems can be solved by preparing a carrier which includes an amino group-containing compound layer and a polyvalent carboxylic acid layer stacked in this order on a resin substrate, in which the resin substrate used contains an inorganic pigment and the resin substrate has a centerline surface average roughness controlled at a predetermined value. This finding has led to the completion of the present invention.

The present disclosure provides a carrier for bio-related molecule immobilization comprising: a resin substrate; an amino group-containing compound layer formed on the resin substrate; and a polyvalent carboxylic acid layer formed on the amino group-containing compound layer, wherein a carboxyl group of the polyvalent carboxylic acid layer is subjected to active esterification, wherein the resin substrate contains an inorganic pigment, and wherein the resin substrate has a centerline surface average roughness Ra of 60 nm or less.

In addition, the present disclosure provides a method of producing a carrier for bio-related molecule immobilization, comprising: providing a resin substrate containing an inorganic pigment and having a centerline surface average roughness Ra of 60 nm or less; forming an aminoalkylsilane layer on the resin substrate; forming a polyvalent carboxylic acid layer on the aminoalkylsilane layer; and subjecting a carboxyl group of the polyvalent carboxylic acid layer to active esterification.

Specifically, in a first and second aspect of the present disclosure, there is provided a carrier for bio-related molecule immobilization and a method of producing a carrier for bio-related molecule immobilization in accordance with the claims appended hereto.

As described above, in the present disclosure, the background value at the time of detecting bio-related molecules can be decreased to obtain a carrier with a high detection sensitivity by using a resin substrate containing an inorganic pigment and controlling the centerline surface average roughness of the resin substrate at a predetermined value.

### Brief Description of Drawings

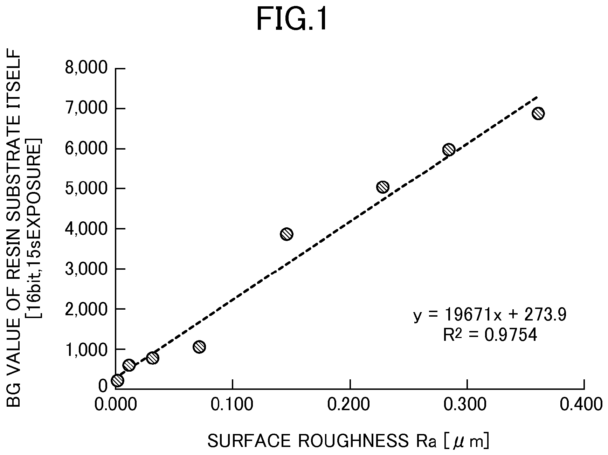

Fig. 1 illustrates a graph created by plotting centerline surface average roughnesses Ra and BG values for blank plates of Reference Examples 1 to 3 and Reference Comparative Examples 1 to 5.
Fig. 2 illustrates spot observation images for carriers of Comparative Examples 1 and 2 and Examples 1 and 2.

### Detailed Description

In accordance with the claims appended hereto, the present disclosure provides a carrier for bio-related molecule immobilization comprising: a resin substrate; an amino group-containing compound layer formed on the resin substrate; and a polyvalent carboxylic acid layer formed on the amino group-containing compound layer, wherein a carboxyl group of the polyvalent carboxylic acid layer is subjected to active esterification, the resin substrate contains an inorganic pigment, and the resin substrate has a centerline surface average roughness Ra of 60 nm or less.

The present invention uses a resin substrate. Although the type of resin is not particularly limited, it is preferable to use a material having as low autofluorescence as possible because the detection of bio-related molecules such as nucleic acids is often carried out based on fluorescent substances bound to the bio-related molecules. Specifically, the types of resin include polyethylene, polypropylene, cyclic polyolefin, polyisobutylene, polyethylene terephthalate, polymethyl methacrylate, polymethyl pentene, unsaturated polyester, fluorine-containing resins, polyvinyl chloride, polyvinylidene chloride, polyvinyl acetate, polyvinyl alcohol, polyvinyl acetal, acrylic resins, polyacrylonitrile, polystyrene, acetal resins, polycarbonate, polyamide, phenolic resins, urea-formaldehyde resins, epoxy resins, melamine resins, styrene-acrylonitrile copolymer, acrylonitrile-butadiene styrene copolymer, and organic materials such as polyphenylene oxide and polysulfone, and a mixture resin of two or more kinds from these may be used. As a material for the resin substrate in the present invention, it is preferable to use a polycarbonate or a polypropylene, and particularly preferable to use a polycarbonate as a material for the resin substrate.

The present invention uses a resin substrate containing an inorganic pigment. As the inorganic pigment, it is possible to use any inorganic pigments such as a black pigment which can decrease the optical transparency of the substrate, among which a black pigment is preferable. Black pigments usable include carbon blacks, carbon nanotubes, aniline black, titanium black, acetylene black, hematite, perylene black, or mixtures thereof. The content of the inorganic pigment in the resin substrate can be determined as appropriate by those skilled in the art, and is preferably 0.1 to 2 parts by weight, more preferably 0.2 to 1 part by weight, and more preferably 0.2 to 0.5 parts by weight relative to 100 parts by weight of the resin. Moreover, for the improvement of detection sensitivity, the resin substrate used in the present disclosure may contain a different substance capable of improving the performance depending on the purpose such as decreasing the BG value, and examples of such a substance include transparent nucleating agents, ultraviolet absorbers, and the like.

In the carrier for bio-related molecule immobilization of the present invention, the resin substrate has a centerline surface average roughness Ra of 60 nm or less, preferably 50 nm or less, more preferably 40 nm or less, further preferably 30 nm or less, further preferably 20 nm or less, and particularly preferably 10 nm or less. In the present invention, the centerline surface average roughness is a numerical value defined by JIS-B-0601-1982, and can be measured with, for example, a contact-type surface roughness/shape measuring machine.

When the type of the above-described resin, the type and the amount blended of the inorganic pigment, and the centerline surface average roughness are adjusted as appropriate, the resin substrate used in the present disclosure has a light transmittance of preferably 80% or more and preferably 90% or more at a wavelength of 450 to 750 nm.

Those skilled in the art can prepare the above resin substrate by any molding method such as injection molding, extrusion molding, and compression molding. Among the above, injection molding and extrusion molding are preferable, and injection molding is particularly preferable.

In the carrier for bio-related molecule immobilization of the present invention, an amino group-containing compound layer is formed on the resin substrate described above. As the amino group-containing compound contained in the amino group-containing compound layer, it is possible to use any compound having one or more unsubstituted or substituted amino groups, and it is possible to use compounds containing ammonia, various amines, amino alcohols, aminoalkylsilanes, and the like, for example. The above amines include allylamine, monomethylamine, dimethylamine, monoethylamine, diethylamine, ethylenediamine, hexamethylenediamine, and n-propylamine. Among the above, the present invention preferably uses an aminoalkylsilane as the amino group-containing compound. For example, the aminoalkylsilane used is one whose alkyl group has 1 to 10 carbon atoms and preferably 2 to 5 carbon atoms, and specifically, the alkyl group can include methyl groups, ethyl groups, propyl groups, butyl groups, and pentyl groups. Among the above, a propyl group is particularly preferable in the present disclosure. In addition, the silane of the aminoalkylsilane may be substituted with one or more substituents. For example, it is possible to use one substituted with an alkoxy group (such as a methoxy group, an ethoxy group, a propoxy group, or a butoxy group) having 1 to 5 and preferably 2 to 4 carbon atoms. It is particularly preferable that the silane be substituted with three ethoxy groups. Specifically, the aminoalkylsilane includes 3-aminopropyltriethoxysilane, 3-aminopropyltrimethoxysilane, 3-aminopropyldiethoxymethylsilane, and 3-aminopropyldimethoxyethylsilane. In the present disclosure, 3-aminopropyltriethoxysilane is particularly preferable. Note that the amino group-containing compound layer may be formed on at least part of the surface of the substrate, and does not need to cover the entire surface of the substrate.

No particular limitation is imposed on the method of forming an amino group-containing compound layer. For example, it is possible to form an amino group-containing compound layer by immersing the substrate in a solution prepared by dissolving the above-described amino group-containing compound in various solvents. The types of solvent used can include alcohols such as methanol and ethanol. For the purpose of reducing the surface roughness of the substrate to suppress autofluorescence, it is preferable to use water as the solvent and immerse the substrate in an aqueous solution in which the amino group-containing compound is sufficiently hydrolyzed.

The immersion time and the concentration of the solution of the amino group-containing compound can be set as appropriate by those skilled in the art in consideration of the type of the specific compound used so as to obtain a predetermined peak intensity ratio of the present disclosure to be described later. For example, it is possible to use a solution of 1% by weight to 10% by weight and preferably 3% by weight to 8% by weight, and it is possible to set the immersion time to 15 minutes to 180 minutes and preferably 30 minutes to 120 minutes.

In the carrier for bio-related molecule immobilization of the present disclosure, a polyvalent carboxylic acid layer is further formed on the aminoalkylsilane layer described above. When the polyvalent carboxylic acid layer is formed in this manner, carboxyl groups are introduced to the surface side of the carrier. No particular limitation is imposed on the type of the polyvalent carboxylic acid used in the present invention. For example, it is possible to use a homopolymer or a copolymer of a monomer having a carboxyl group such as polyacrylic acid, polymethacrylic acid, polymaleic acid, polyitaconic acid, and acrylic acid-methacrylic acid copolymers. When the carboxyl group of the polyvalent carboxylic acid forms an amide bond with the amino group of the aminoalkylsilane layer, the polyvalent carboxylic acid layer can be firmly bound on the aminoalkylsilane layer. Note that the polyvalent carboxylic acid layer may be formed on at least part of the surface of the underlying substrate and/or the aminoalkylsilane layer, and does not need to cover the entire surface of the substrate and/or the aminoalkylsilane layer.

No particular limitation is imposed on the method of forming a polyvalent carboxylic acid layer. The method includes a method of immersing the substrate having an aminoalkylsilane layer formed thereon in a solution of the polyvalent carboxylic acid. The solvent used for the solution of the polyvalent carboxylic acid can be selected as appropriate by those skilled in the art, and it is possible to use water and various types of organic solvents including alcohols such as methanol and ethanol. In the present disclosure, it is preferable to use an aqueous solution.

The immersion time, the concentration, and the molecular weight of the solution of the polyvalent carboxylic acid layer can be set as appropriate by those skilled in the art. For example, the molecular weight selected is 25,000 to 1,000,000, preferably 50,000 to 500,000, and particularly preferably 100,000 to 200,000, the concentration selected is 0.1% by weight to 10% by weight, preferably 0.5% by weight to 10% by weight, and particularly preferably 1.0% by weight to 5.0% by weight, and the immersion time selected is 1 minute to 60 minutes, preferably 5 minutes to 30 minutes, and particularly preferably 10 minutes to 20 minutes.

In the carrier for bio-related molecule immobilization of the present invention, the carboxyl groups of the polyvalent carboxylic acid layer formed as described above are subjected to active esterification. When the carboxyl groups are subjected to active esterification to form active ester groups, it is possible to stably immobilize bio-related molecules at the time of eventually spotting a solution of bio-related molecules as a carrier for bio-related molecule immobilization. As regards the type of active ester group and a method of forming the same, there is no particular limitation thereon and those skilled in the art can appropriately select ones suitable for the application as a carrier for bio-related molecule immobilization. The active ester group includes nitrophenyl groups, N-hydroxysuccinimide groups, N-hydroxynorbornene-2,3-dicarboximide groups, succinimide groups, and phthalimide groups. In the present disclosure, N-hydroxysuccinimide groups are preferable. The method of forming active ester groups includes active esterification of the carboxyl groups of the polyvalent carboxylic acid layer by immersion in a solution prepared by dissolving a dehydration condensation agent such as 1-ethyl-3-(3-dimethylaminopropyl)carbodiimide hydrochloride and any type of electrophilic group introducing agent corresponding to the active ester group as described above (such as N-hydroxysuccinimide) in a buffer solution.

The carrier for bio-related molecule immobilization of the present invention obtained as described above can immobilize bio-related molecules on its surface. The bio-related molecules in the present disclosure are preferably nucleic acids. A nucleic acid-containing solution is spotted on a carrier for bio-related molecule immobilization, followed by washing of the unreacted nucleic acid solution not bound on the carrier. Thus, it is possible to obtain a carrier with immobilized nucleic acids. The method of spotting a nucleic acid-containing solution on the carrier includes, but is not particularly limited to, a spotting method including bringing a pin holding a nucleic acid-containing solution into contact with the carrier and a method of spraying by ink-jet a nucleic acid-containing solution on the carrier. It is possible to carry out spotting using any apparatus, method, and the like known to those skilled in the art.

The nucleic acid immobilization carrier prepared as described above can be used for detecting the presence of target nucleic acids in the test sample. For example, consider the case of using DNA as a nucleic acid. DNA is extracted from the test sample and amplified, which is hybridized with nucleic acids on a nucleic acid immobilization carrier (such as a DNA chip or a microarray) for detection. This makes it possible to confirm the presence or absence of specific microbial contamination in the test sample. The method of extracting DNA includes the phenol extraction method, the phenol-chloroform extraction method, the alkali dissolution method, and the boiling method. Examples also include a method of extracting DNA using a commercially available DNA extracting reagent or a nucleic acid automatic extraction apparatus.

The target region of the extracted DNA is amplified by a nucleic acid amplification method, if necessary. The target region is a region of chromosomal DNA which can be amplified by the nucleic acid amplification method, and can be set as appropriate depending on the purpose without particular limitation as long as it is possible to detect the detection target microorganism. For example, when the test sample contains cells different in type from the detection target microorganism, the target region preferably has a sequence specific to the detection target microorganism, or may have a sequence common to two or more types of microorganisms depending on the purpose. The nucleic acid amplification method includes the PCR method (polymerase chain reaction), the SDA method (strand displacement amplification), the LCR method (ligase chain reaction), the LAMP method (loop-mediated isothermal amplification), and the ICAN method (isothermal and chimeric primer-initiated amplification of nucleic acids). Among these, it is preferable to use the PCR method. For example, the length of a target region amplified by the PCR method is usually 80 to 1000 bases and preferably 100 to 500 bases.

The amplified DNA is detected with the nucleic acid immobilization carrier of the present disclosure. The nucleic acids (probes) immobilized on the carrier are detectors which enable detection by binding only to the target bio-related molecules in the case where various bio-related molecules such as specific genes and proteins are coexistent, and it is thus difficult to make a distinguishment from one another and to make a direct selection. For example, consider the case of detecting the nucleic acid of a specific microorganism as a bio-related molecule. The probes used are DNA fragments having a sequence complementary to the base sequence possessed by the nucleic acid of this microorganism, and hybridization with the nucleic acid is carried out. Usually, DNAs of 1 to 200 bases and preferably 10 to 150 bases are immobilized on the probes. Either single stranded or double stranded DNA can be immobilized. In addition, when the target bio-related molecules are labeled with a fluorescent substance or the like in advance, it is possible to detect the bio-related molecules bound to the probe. The solution used for the binding reaction between the bio-related molecules and the probes contains, for example, bio-related molecules as well as a buffer solution prepared by adding SDS (sodium dodecyl sulfate) to citric acid-saline.

Hereinafter, the teachings of the present disclosure are described in more detail based on Examples.

### [Examples]

### 1. Relationship between Surface Roughness of Resin Substrate and BG Value at Time of Detection

A polycarbonate containing carbon black in an amount of 0.5% was used to prepare by injection molding substrates having different surface roughnesses Ra. Each blank plate before surface treatment was photographed with a DNA chip detection apparatus GENOGATE Reader (manufactured by Toyo Seikan Group Holdings, Ltd.). The photographing conditions were such that a dry blank plate was irradiated with a laser ray of 640 nm and photographed with a 16-bit monochrome camera (luminance range: 0 (black) to 65536 (white)) with an exposure time of 15 [s] to measure the average value of the image luminances of three points of each blank plate at that time as a background value (BG value). Table 1 presents the results.

**Table 1**

| | Surface Roughness Ra [µm] | Luminance of Photographed Image (Average Value) |
|---|---|---|
| Reference Comparative Example 1 | 0.360 | 6,888 |
| Reference Comparative Example 2 | 0.284 | 5,986 |
| Reference Comparative Example 3 | 0.227 | 5,057 |
| Reference Comparative Example 4 | 0.146 | 3,872 |
| Reference Comparative Example 5 | 0.071 | 1,070 |
| Reference Example 1 | 0.032 | 791 |
| Reference Example 2 | 0.012 | 612 |
| Reference Example 3 | 0.002 | 222 |

### 2. Preparation of DNA Immobilization Carrier

A polycarbonate containing carbon black in an amount of 0.5% was used to prepare substrates having different surface roughnesses Ra. The BG value of each blank plate was measured in the same manner as that of 1 above.

After that, the resin substrate was immersed in a 5wt% aqueous solution of 3-aminopropyltriethoxysilane for 30 minutes to introduce amino groups. This substrate, into which amino groups were introduced, was immersed in a 1wt% aqueous solution of polyacrylic acid for 10 minutes, then washed with pure water, and immersed for 10 minutes in an activation solution prepared by dissolving 100 mM of 1-ethyl-3-(3-dimethylaminopropyl)carbodiimide hydrochloride and 50 mM of N-hydroxysuccinimide in a 0.1 M phosphate buffer solution (pH 6.8), to thereby obtain a carrier having activated carboxyl groups.

A DNA probe solution prepared to 10 mM using a microarray preparation apparatus was spotted on the carrier. The substrate on which the DNA probe was spotted was heated in an oven at 80°C for 1 hour, and then washed with 2 × SSC/0.2% SDS at room temperature for 10 minutes and at 60°C for 10 minutes to prepare a DNA immobilization substrate.

### 3. Evaluation of Detection Sensitivity of Nucleic Acid Immobilization Carrier

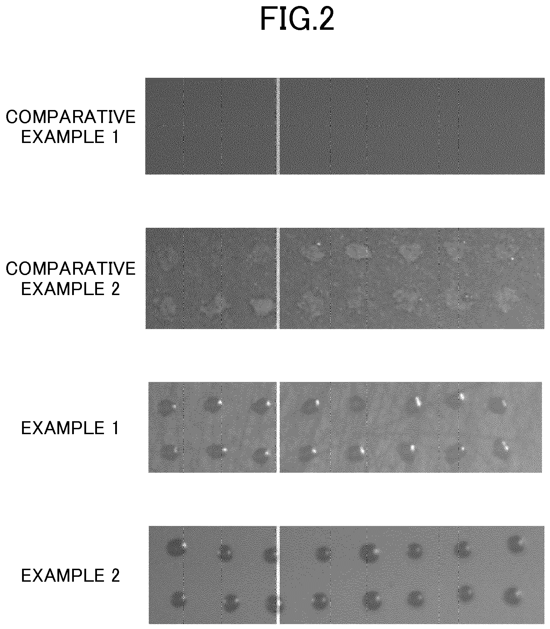

A hybridization buffer in an amount of 40 ml and probe-complementary DNA were mixed at 0.125 nM to come into contact with the surface of the prepared DNA immobilization carrier, followed by reaction at 45°C for 60 minutes. After the reaction, the substrate was subjected to sway washing with a 0.5 × SSC/0.2% SDS solution and then with a 0.5 × SSC solution twice for 50 cycles each. A cover glass was placed thereon, and GENOGATE Reader (manufactured by Toyo Seikan Group Holdings, Ltd.) was used to obtain a fluorescence detection image. The S/N ratio ((fluorescence intensity - BG value)/BG value) was calculated from the fluorescence intensity value and the background value obtained from each of the spots on the detection image. The DNA chip measurement prepared four carriers having four 16-spot sets for each of Examples and Comparative Examples, and determined the average value measured for a total of 64 spots. Fig. 2 illustrates images after carrier spotting, and Table 2 presents the spot characteristics and the measurement results at the time of detection.

**Table 2**

| | Surface Roughness Ra [µm] | Blank Plate BG Value | Spot Appropriateness (Spot Shape) | DNA Chip Measurement | | | |
|---|---|---|---|---|---|---|---|
| | | | | Fluorescence Intensity | BG Value | S/N Ratio | Determination |
| Comparative Example 1 | 0.360 | 6520 | × (Inappropriate) | Not Measured | Not Measured | Not Measured | × |
| Comparative Example 2 | 0.181 | 3872 | Δ (Partial Blurring) | 8522 | 1295 | 5.6 | Δ |
| Example 1 | 0.059 | 1052 | ○ (Good) | 9724 | 497 | 18.6 | ○ |
| Example 2 | 0.002 | 186 | ○ (Good) | 8774 | 267 | 32.2 | ⊚ |

It is understood from the above results that it is possible to obtain a carrier having a good spot shape and a low BG value when the surface roughness is in the predetermined range of the present disclosure.

## Claims

1. A carrier for bio-related molecule immobilization comprising:
a resin substrate;
an amino group-containing compound layer formed on the resin substrate; and
a polyvalent carboxylic acid layer formed on the amino group-containing compound layer,
wherein a carboxyl group of the polyvalent carboxylic acid layer is active esterified,
wherein the resin substrate contains an inorganic pigment,
wherein the resin substrate has a centerline surface average roughness Ra as defined according to JIS-B-0601-1982 of 60 nm or less, and
wherein the resin of the resin substrate is a polycarbonate or a polypropylene.

2. The carrier for bio-related molecule immobilization according to claim 1, wherein the resin substrate has a centerline surface average roughness Ra of 10 nm or less.

3. The carrier for bio-related molecule immobilization according to claim 1 or 2, wherein the inorganic pigment is a black pigment.

4. The carrier for bio-related molecule immobilization according to claim 3, wherein the black pigment is selected from the group consisting of carbon blacks, carbon nanotubes, aniline black, titanium black, acetylene black, hematite, perylene black, and mixtures thereof.

5. The carrier for bio-related molecule immobilization according to any one of claims 1 to 4, wherein a content of the inorganic pigment in the resin substrate is 0.1 to 5 parts by weight relative to 100 parts by weight of a resin.

6. A method of producing a carrier for bio-related molecule immobilization, comprising:
providing a resin substrate containing an inorganic pigment and having a centerline surface average roughness Ra as defined according to JIS-B-0601-1982 of 60 nm or less, wherein the resin of the resin substrate is a polycarbonate or a polypropylene;
forming an aminoalkylsilane layer on the resin substrate;
forming a polyvalent carboxylic acid layer on the aminoalkylsilane layer; and
subjecting a carboxyl group of the polyvalent carboxylic acid layer to active esterification.

7. The method according to claim 6, wherein the resin substrate is formed by injection molding or extrusion molding.

## Patentansprüche

1. Träger zur Immobilisierung biobezogener Moleküle, umfassend:
ein Harzsubstrat;
eine aminogruppenhaltige Verbindungsschicht, die auf dem Harzsubstrat gebildet ist; und
eine mehrwertige Carbonsäureschicht, die auf der aminogruppenhaltigen Verbindungsschicht gebildet ist,
wobei eine Carboxylgruppe der mehrwertigen Carbonsäureschicht aktiv verestert ist,
wobei das Harzsubstrat ein anorganisches Pigment enthält,
wobei das Harzsubstrat einen arithmetischen Mittenrauwert Ra wie in JIS-B-0601-1982 definiert von 60 nm oder weniger aufweist, und
wobei das Harz des Harzsubstrats ein Polycarbonat oder ein Polypropylen ist.

2. Träger zur Immobilisierung biobezogener Moleküle nach Anspruch 1, wobei das Harzsubstrat einen arithmetischen Mittenrauwert Ra von 10 nm oder weniger aufweist.

3. Der Träger zur Immobilisierung biobezogener Moleküle nach Anspruch 1 oder 2, wobei das anorganische Pigment ein schwarzes Pigment ist.

4. Träger zur Immobilisierung biobezogener Moleküle nach Anspruch 3, wobei das schwarze Pigment ausgewählt ist aus der Gruppe bestehend aus Ruß, Kohlenstoffnanoröhren, Anilinschwarz, Titanschwarz, Acetylenschwarz, Hämatit, Perylenschwarz und Mischungen davon.

5. Träger zur Immobilisierung biobezogener Moleküle nach einem der Ansprüche 1 bis 4, wobei ein Gehalt an anorganischem Pigment im Harzsubstrat 0,1 bis 5 Gewichtsteile bezogen auf 100 Gewichtsteile Harz beträgt.

6. Verfahren zur Herstellung eines Trägers zur Immobilisierung biobezogener Moleküle, umfassend:
Bereitstellen eines Harzsubstrats, das ein anorganisches Pigment enthält und einen arithmetischen Mittenrauwert Ra wie in JIS-B-0601-1982 definiert von 60 nm oder weniger aufweist, wobei das Harz des Harzsubstrats ein Polycarbonat oder ein Polypropylen ist;
Bilden einer Aminoalkylsilanschicht auf dem Harzsubstrat;
Bilden einer mehrwertigen Carbonsäureschicht auf der Aminoalkylsilanschicht; und
Unterziehen einer Carboxylgruppe der mehrwertigen Carbonsäureschicht einer aktiven Veresterung.

7. Verfahren nach Anspruch 6, wobei das Harzsubstrat durch Spritzgießen oder Extrusionsformen gebildet wird.

## Revendications

1. Support pour l'immobilisation de molécules biologiques comprenant :
un substrat en résine ;
une couche de composé contenant un groupe amino formée sur le substrat en résine ; et
une couche d'acide carboxylique polyvalent formée sur la couche de composé contenant le groupe amino,
dans lequel un groupe carboxyle de la couche d'acide carboxylique polyvalent est estérifié de manière active,
dans lequel le substrat en résine contient un pigment inorganique,
dans lequel le substrat en résine présente une rugosité moyenne Ra de surface de la ligne centrale, telle que définie selon la norme JIS-B-0601-1982, inférieure ou égale à 60 nm, et
dans lequel la résine du substrat en résine est un polycarbonate ou un polypropylène.

2. Support pour l'immobilisation de molécules biologiques selon la revendication 1, dans lequel le substrat en résine présente une rugosité moyenne Ra de surface de la ligne centrale inférieure ou égale à 10 nm.

3. Support pour l'immobilisation de molécules biologiques selon la revendication 1 ou 2, dans lequel le pigment inorganique est un pigment noir.

4. Support pour l'immobilisation de molécules biologiques selon la revendication 3, dans lequel le pigment noir est choisi parmi le groupe consistant en noirs de carbone, nanotubes de carbone, noir d'aniline, noir de titane, noir d'acétylène, hématite, noir de pérylène et des mélanges de ceux-ci.

5. Support pour l'immobilisation de molécules biologiques selon l'une quelconque des revendications 1 à 4, dans lequel la teneur en pigment inorganique dans le substrat en résine va de 0,1 à 5 parties en poids par rapport à 100 parties en poids d'une résine.

6. Procédé de production d'un support pour l'immobilisation de molécules biologiques, comprenant :
la fourniture d'un substrat en résine contenant un pigment inorganique et présentant une rugosité moyenne Ra de surface de la ligne centrale, telle que définie selon la norme JIS-B-0601-1982, inférieure ou égale à 60 nm, dans lequel la résine du substrat en résine est un polycarbonate ou un polypropylène ;
la formation d'une couche d'aminoalkylsilane sur le substrat en résine ;
la formation d'une couche d'acide carboxylique polyvalent sur la couche d'aminoalkylsilane ; et
la soumission d'un groupe carboxyle de la couche d'acide carboxylique polyvalent à une estérification active.

7. Procédé selon la revendication 6, dans lequel le substrat en résine est formé par moulage par injection ou par moulage par extrusion.
